Europäisches Patentamt

European Patent Office

Office européen des brevets

(11) Publication number: **0 292 131**
**B1**

(12) **EUROPEAN PATENT SPECIFICATION**

(45) Date of publication of patent specification: **06.02.91**

(51) Int. Cl.⁵: **C 07 D 307/89** // B41M1/00

(21) Application number: **88303882.0**

(22) Date of filing: **28.04.88**

(54) Prevention of sludge formation.

(30) Priority: **07.05.87 GB 8710820**

(43) Date of publication of application:
**23.11.88 Bulletin 88/47**

(45) Publication of the grant of the patent:
**06.02.91 Bulletin 91/06**

(84) Designated Contracting States:
**BE DE FR GB IT NL SE**

(56) References cited:
**EP-A-0 066 436**
**EP-A-0 083 224**
**DE-A-3 418 057**

(73) Proprietor: **BP Chemicals Limited
Belgrave House 76 Buckingham Palace Road
London, SW1W 0SU (GB)**

(72) Inventor: **Mytton, Roy BP Chemicals Limited
Speciality Chemicals Division 84 Mill Lane
Carshalton Surrey SM5 2JT (GB)**

(74) Representative: **Krishnan, Suryanarayana
Kalyana et al
BP INTERNATIONAL LIMITED Patents &
Agreements Division Chertsey Road
Sunbury-on-Thames Middlesex TW16 7LN (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

The present invention relates to a method of preventing sludge formation in trimellitic anhydride solutions during transportation, storage or use.

Solutions of trimellitic anhydride (hereafter referred to as "TMA") are prone to sludge formation due to the limited solubility of the anhydride in conventional solvents. Moreover, even clear solutions which may be obtained by separation of the sludge from the solution can upon storage become cloudy with further sludge formation. This recurrence of sludge formation is primarily due for instance to:

(a) failure to totally remove the particulate suspension from the previously treated sludge, and/or

(b) ingress of moisture into the solution which results in the hydrolysis of the anhydride to the corresponding carboxylic acid which is relatively insoluble.

These problems are especially noticeable when the solutions are stored at ambient temperature. Moreover, the sludge once formed is in very fine particulate form which therefore results in the formation of a colloidal suspension. Such colloidal suspensions are stable and hence not only difficult to filter but also prevent the particulate suspension from settling down as a sediment within a reasonable duration at the bottom of the container where the solution is stored.

Solutions of TMA in butanone or cyclohexanone are used by PVC processors operating a print inhibition process. TMA is an effective inhibitor preventing the decomposition of azodicarbonamide blowing agents which are used in the formation of cellular PVC.

Handling solid TMA can give rise to hazardous airborne dust and solutions offer a convenient way of avoiding this hazard. Conventionally a phthalate ester (e.g. Bisoflex DL79P® a BP Chemicals product) is incorporated in the TMA solution at 2.5% w/w as a dampening agent to prevent dusting once the solvent has been removed in usage. However, this ester does not prevent the formation of sludge in the solution during storage.

Thus, it has always been necessary not only to use a dampening agent but also a means of preventing sludge formation in such solutions of TMA.

Expedients such as the use of stronger solvents have been impractical because additives containing nitrogen, sulphur, phosphorus and the like interfere with the print inhibition process for which the solution is primarily intended. A typical colloid of TMA was found to contain the following major components upon NMR and GLC analysis in spite of using 2.5% w/w of the phthalate ester in the solutions: TMA, trimellitic acid and phthalic acids.

It has now been found that these problems of sludge formation and colloid can be mitigated to a substantial extent by treating the solution with a specific class of coagulants followed by separation of the suspended solids.

Accordingly, the present invention is a composition comprising trimellitic anhydride, a ketonic solvent and a coagulant selected from a di- or polyhydric alcohol having a molecular weight of at least 100 and a fatty acid ester of said di- or polyhydric alcohol.

According to a further embodiment of the present invention is a process for producing a substantially clear and stable solution of trimellitic anhydride in a ketonic solvent said process comprising treating a colloidal suspension of the anhydride in the solvent with a coagulant in order to coagulate the suspended particulate matter in the colloid followed by removal of said particulate matter from the clear solution characterised in that the coagulant is a di- or polyhydric alcohol having a molecular weight of at least 100 or a fatty acid ester of said di- or polyhydric alcohol.

The ketonic solvent for TMA is suitably butanone (also known as methyl ethyl ketone) or cyclohexanone because TMA has a substantially high solubility in these solvents under ambient conditions. By ambient conditions is meant a temperature from 5—25°C, suitably 5—20°C.

The di- or polyhydric alcohol used is suitably a liquid and has a molecular weight in the range from 100—750, preferably from 100—600.

Specific examples of such alcohols include the di- and polyoxyalkylene glycols especially diethylene glycol, triethylene glycol, polyethylene glycols, sorbitol and the like. The fatty acid esters are suitably the oleates, stearates and laurates, especially the mono-laurate and the mono- and tri-oleates of sorbitol and polyethylene glycol.

The coagulant is suitably added to the colloidal suspension of TMA in an amount from 1—5% w/w of the total trimellitic anhydride in the suspension, suitably from 2—3% w/w of the total TMA.

By the term "colloidal suspension" as herein used is meant throughout the specification that the solution of TMA contains suspended finely divided particulate matter which does not readily settle down as a sediment upon standing and is not easily filtered.

It is a feature of the present invention that the use of the coagulants now specified obviates the need to use a separate dampening agent. Furthermore, once the suspended solids have been removed after coagulation, the resultant clear solution is stable and does not become cloudy even after prolonged storage.

In addition, the coagulants now used enable the colloidal suspension to be broken more readily so much so that the clear solution can be separated from the particulate matter by simple decantation.

Thus, according to yet another embodiment, the present invention is a clear stable solution of TMA in a ketonic solvent containing a stabilizing amount of a di- or polyhydric alcohol having a molecular weight of at least 100 or a fatty acid ester of said di- or polyhydric alcohol.

The present invention is further illustrated with reference to the following Examples.

Examples

A series of TMA solutions were prepared using coagulants at a concentration of 2.5% w/w based on the total solution. The solution of TMA was prepared using cyclohexanone as the solvent and the coagulant was added after the TMA solution has been stirred for 15 minutes at ambient temperature.

GLC analysis of stock TMA used (ex Amoco, analysed as their methyl ester) for the preparation of all solutions gave the following approximate compositions:

|  | % w/w |
|---|---|
| Trimellitic anhydride+acid | 98.2 |
| Terephthalic acid | 0.5 |
| Isophthalic acid | 0.5 |
| Phthalic acid | 0.2 |
| Pyromellitic acid/anhydride | 0.3 |
| Unknown | 0.3 |

Note: Acid and anhydride are indistinguishable by this method.

The coagulants were:—
Diethylene glycol
Trimethylene glycol
Polyethylene glycol 200
Sorbitan monooleate (SPAN* 80S)
Sorbitan trioleate (SPAN* 85S)
Sorbitan monolaurate (SPAN* 20S)
Polyethylene glycol monolaurate (NONEX* 139) (G)
Polyethylene glycol monooleate (NONEX* 30) (H)

*Registered trade mark

The ease of coagulation in the following table was visually monitored.

Results

| Additive used | Coagulation effect | Comments | Upon storage* |
|---|---|---|---|
| None | Nil | — | — |
| Diethylene glycol | +ve | Decantible after 24 hr | — |
| Triethylene glycol | +ve | " " | Trace of ppt |
| Polyethylene glycol 200 | +ve | " " | No ppt |
| SPAN 80S (sorbitan monoleate) | +ve | " " | " " |
| SPAN 85S (sorbitan trioleate) | +ve | " " | — |
| SPAN 20S (sorbitan monolaurate) | +ve | " " | Trace of ppt |
| NONEX 139 (PEG 400 monolaurate) | sl+ve | Decantible after 96 hr | — |
| NONEX 30 (PEG 400 monoleate) | sl+ve | " " | — |

*For 4 weeks at 5°C to test thermal stability of solution.

3

The above results show that using coagulants of the present invention, the suspension if any formed, is readily coagulated into a sediment which can be decanted and the remaining clear solution is stable upon storage.

The samples noted above were tested for their dustiness upon evaporation to assess the dampening effect of the coagulants used. Upon evaporation at 130°C for 1 hour in an oven, the clear and yellow solutions of TMA containing the coagulant of the present invention resulted in residues which were either treacly or waxy in appearance.

## Claims

1. A composition comprising trimellitic anhydride, a ketonic solvent and a coagulant selected from a di- or polyhydric alcohol having a molecular weight of at least 100 and a fatty acid ester of said di- or polyhydric alcohol.

2. A composition according to claim 1 wherein the ketonic solvent is one in which trimellitic anhydride has a substantially high solubility under ambient conditions.

3. A composition according to claim 1 or 2 wherein the ketonic solvent is methyl ethyl ketone.

4. A composition according to claim 1 or 2 wherein the di- or polyhydric alcohol used in the coagulant is a liquid having a molecular weight in the range from 100—750.

5. A composition according to any one of the preceding claims wherein the di- or polyhydric alcohol is selected from diethylene glycol, triethylene glycol polyethylene glycols and sorbitol.

6. A composition according to any one of the preceding claims wherein the fatty acid ester of the di- or polyhydric alcohol is an oleate, stearate or a laurate.

7. A composition according to claim 6 wherein the fatty acid ester is selected from the mono-laurate, mono-oleate and mono-stearate of sorbitol and polyethylene glycol.

8. A composition according to any one of the preceding claims wherein the coagulant is present in the composition in an amount from 1—5% w/w of the total of trimellitic anhydride in the composition.

9. A process for producing a substantially clear and stable solution of trimellitic anhydride in a ketonic solvent said process comprising treating a colloidal suspension of the anhydride in the solvent with a coagulant in order to coagulate the suspended particulate matter in the colloid followed by removal of said particulate matter from the clear solution characterised in that the coagulant is a di- or polyhydric alcohol having a molecular weight of at least 100 or a fatty acid ester of said di- or polyhydric alcohol.

10. A clear stable solution of trimellitic anhydride in a ketonic solvent containing a stabilising amount of a di- or polyhydric alcohol having a molecular weight of at least 100 or a fatty acid ester of said di- or polyhydric alcohol.

## Patentansprüche

1. Zusammensetzung umfassend Trimellithsäureanhydrid, ein ketonartiges Lösungsmittel und ein Gerinnungsmittel ausgewählt aus einem zwei- oder mehrwertigem Alkohol mit einem Molekulargewicht von mindestens 100 und einem Fettsäureester von diesem zwei- oder mehrwertigen Alkohol.

2. Zusammensetzung nach Anspruch 1, worin das ketonartige Lösungsmittel eines ist, in dem das Trimellithsäureanhydrid eine im wesentlichen hohe Löslichkeit unter Umgebungsbedingungen hat.

3. Zusammensetzung nach Anspruch 1 oder 2, worin das ketonartige Lösungsmittel Methylethylketon ist.

4. Zusammensetzung nach Anspruch 1 oder 2, worin der im Gerinnungsmittel benutzte zwei- oder mehrwertige Alkohol eine Flüssigkeit ist, die ein Molekulargewicht im Bereich von 100—750 hat.

5. Zusammensetzung nach einem der vorstehenden Ansprüche, worin der zwei- oder mehrwertige Alkohol ausgewählt ist aus Diethylenglykol, Triethylenglykol, Polyethylenglykolen und Sorbit.

6. Zusammensetzung nach einem der vorstehenden Ansprüche, worin der Fettsäureester des zwei- oder mehrwertigen Alkohols ein Oleat, Stearat oder ein Laurat ist.

7. Zusammensetzung nach Anspruch 6, worin der Fettsäureester ausgewählt ist aus Monolaurat, Monooleat und Monostearat von Sorbit und Polyethylenglykol.

8. Zusammensetzung nach einem der vorstehenden Ansprüche, worin das Gerinnungsmittel in der Zusammensetzung vorhanden ist in einer Menge von 1—5% Gew./Gew. des gesamten Trimellithsäureanhydrids in der Zusammensetzung.

9. Verfahren zur Herstellung einer im wesentlichen klaren und stabilen Lösung von Trimellithsäureanhydrid in einem ketonartigen Lösungsmittel, wobei dieses Verfahren das Behandeln einer kolloidalen Suspension des Anhydrids im Lösungsmittel mit einem Gerinnungsmittel, um die suspendierte teilchenförmige Substanz im Kolloid zu gerinnen, gefolgt von dem Entfernen dieser teilchenförmigen Substanz aus der klaren Lösung umfaßt, dadurch gekennzeichnet, daß das Gerinnungsmittel ein zwei- oder mehrwertiger Alkohol mit einem Molekulargewicht von mindestens 100 oder ein Fettsäureester dieses zwei- oder mehrwertigen Alkohols ist.

10. Klare, stabile Lösung von Trimellithsäureanhydrid in einem ketonartigen Lösungsmittel, das eine stabilisierende Menge eines zwei- oder mehrwertigen Alkohols mit einem Molekulargewicht von mindestens 100 oder eines Fettsäureesters dieses zwei oder mehrwertigen Alkohols enthält.

**Revendications**

1. Composition comportant de l'anhydride trimellitique, un solvant cétonique et un coagulant choisi parmi un alcool di- ou polyhydrique d'un poids moléculaire d'au moins 100 et un ester d'acide gras dudit alcool di- ou polyhydrique.

2. Composition selon la revendication 1, dans laquelle le solvant cétonique est un solvant dans lequel l'anhydride trimellitique présente une solubilité sensiblement élevée dans les conditions ambiantes.

3. Composition selon la revendication 1 ou 2, dans laquelle le solvant cétonique est la méthyléthylcétone.

4. Composition selon la revendication 1 ou 2, dans laquelle l'alcool di- ou polyhydrique utilisé dans le coagulant est un liquide d'un poids moléculaire de 100 à 750.

5. Composition selon l'une quelconque des revendications précédentes, dans laquelle on choisit l'alcool di- ou polyhydrique parmi le diéthylèneglycol, le triéthylèneglycol, les polyéthylèneglycols et le sorbitol.

6. Composition selon l'une quelconque des revendications précédentes, dans laquelle l'ester d'acide gras de l'alcool di- ou polyhydrique est un oléate, un stéarate ou un laurate.

7. Composition selon la revendication 6 dans laquelle on choisit l'ester d'acide gras parmi le mono-laurate, le mono-oléate et le mono-stéarate de sorbitol et de polyéthylèneglycol.

8. Composition selon l'une quelconque des revendications précédentes, dans laquelle le coagulant est présent dans la composition dans une proportion de 1—5% en poids du total de l'anhydride trimellitique dans la composition.

9. Procédé de production d'une solution sensiblement claire et stable d'anhydride trimellitique dans un solvant cétonique, ledit procédé consistant à traiter une solution colloïdale de l'anhydride dans le solvant avec un coagulant pour faire coaguler la matière particulaire en suspension dans le colloïde, puis à éliminer ladite matière particulaire de la solution claire, caractérisé en ce que le coagulant est un alcool di- ou polyhydrique d'un poids moléculaire d'au moins 100 ou un ester d'acide gras dudit alcool di- ou polyhydrique.

10. Solution stable claire d'anhydride trimellitique dans un solvant cétonique contenant une proportion stabilisante d'alcool di- ou polyhydrique d'un poids moléculaire d'au moins 100 ou d'un ester d'acide gras dudit alcool di- ou polyhydrique.